# EUROPÄISCHE PATENTSCHRIFT

(11) **EP 3 723 654 B1**
(45) Veröffentlichungstag und Bekanntmachung des Hinweises auf die Patenterteilung: **09.03.2022**
(21) Anmeldenummer: 18822299.6
(22) Anmeldetag: 11.12.2018
(51) Int. Cl.: A61B 34/10, A61F 2/28, A61B 17/00

(54) **OPERATIONSPLANUNGSSYSTEM FÜR DIE REKONSTRUKTION VON FEHLENDEN ODER GESCHÄDIGTEN KNOCHENTEILEN**
OPERATION PLANNING SYSTEM FOR THE RECONSTRUCTION OF MISSING OR DAMAGED BONE PARTS
SYSTÈME DE PLANIFICATION D'OPÉRATION POUR LA RECONSTRUCTION DE PARTIES OSSEUSES MANQUANTES OU LÉSÉES

(30) Priorität: 11.12.2017 DE 102017222368
(43) Veröffentlichungstag der Anmeldung: 21.10.2020
(73) Patentinhaber: Rheinisch-Westfälische Technische Hochschule (RWTH) Aachen, 52056 Aachen (DE)
(72) Erfinder: MODABBER, Ali, 52074 Aachen (DE); RAITH, Stefan, 94360 Mitterfels (DE)
(74) Vertreter: RCD Patent Giesen, Schmelcher & Griebel Patentwanwälte PartG mbB
(86) Internationale Anmeldenummer: PCT/EP2018/084264
(87) Internationale Veröffentlichungsnummer: WO 2019/115486

(56) Entgegenhaltungen:
- WO-A1-2013/087082
- WO-A1-2015/081232
- WO-A1-2017/162444
- US-A1- 2013 296 872

## Beschreibung

Die Rekonstruktion von Knochen bzw. von Knochen und Gewebeteilen - sei es zur Wiederherstellung eines kosmetischen Ergebnisses und/oder sei es zur Wiederherstellung einer Funktion - ist ein immer wieder auftretender Fall in der Medizin.

Gründe für die Notwendigkeit einer Wiederherstellung können z.B. darin gefunden werden, dass Gewebe und Knochenteile auf Grund von gutartiger oder bösartiger Wucherungen entfernt werden mussten oder aber in Folge eines Unfalls geschädigt oder verloren gegangen sind oder kongenitale Deformitäten aufweisen.

In diesen Fällen wurde in der Vergangenheit bereits auf OP-Planungssysteme zurückgegriffen.

Beispielhaft sei auf die Internationale Patentanmeldung WO 2014 / 188 369 A1 verwiesen, die ein Verfahren zur Planung einer chirurgischen Führung zeigt, bei dem zunächst ein dreidimensionales Bild von mindestens einem Teil eines Spenderknochens bereitgestellt wird. Der Knochen definiert dabei eine äußere dreidimensionale Oberfläche. Es werden eine oder mehrere empfindliche anatomische Strukturen identifiziert, die nicht an der Knochenernte beteiligt sein dürfen. So wird das Volumen des Knochens, das zur Spende geeignet ist, identifiziert, wobei die empfindlichen anatomischen Strukturen ausgeschlossen und das Volumen durch einen Abschnitt der äußeren Oberfläche des Knochens begrenzt wird. Die Parameter für die chirurgische Führung werden im Wesentlichen derart erfasst, dass eine Führungsfläche identifiziert wird.

Dieses Verfahren ist jedoch sehr rechenintensiv und lässt eine schnelle OP-Planung nicht zu. Dies ist unter anderem darin begründet, dass Berechnungen im Volumen vorgenommen werden. Zudem erlaubt dieses Verfahren nicht die Definition von Kurven, die entlang anatomisch relevanter Strukturen verlaufen.

Weiterhin ist das System nicht geeignet den Zielraum geeignet in seiner Vielschichtigkeit zu berücksichtigen.

Zwar ist es seit Ende der 1980 Jahre bekannt computer-assistierte Chirurgie zu verwenden, jedoch sind die bisherigen Verfahren auf Basis von vorgebogenen Platten oder Gewebestrukturen oder Transplantatschnittschablonen zeitaufwändig und vergleichsweise unpräzise. Dennoch sind diese Verfahren gegenüber herkömmlichen chirurgischen Verfahren vorteilhaft, da sie in aller Regel die ischämische Zeit und/oder die Größe des Spenderareals verringern und häufig ein verbessertes funktionales und/oder ästhetisches Ergebnis bereitstellen. Dieses Ergebnis bleibt jedoch immer noch stark abhängig vom Chirurgen und seiner Erfahrung.

Zwar gibt es mittlerweile Softwareansätze, die eine Schritt-für-Schrittführung unterstützen, jedoch ist der Aufwand immer noch enorm, wobei die Operationsplanung lediglich durch die Führung unterstützt wird. Ein solches System ist beispielsweise aus der WO 2013 / 087 082 A1 bekannt. Daher wird im Allgemeinen bei der Operationsplanung ein entsprechend klinisch geschulter Ingenieur benötigt, der in aller Regel im Wege einer gemeinsamen Planung (z.B. über eine Konferenz) involviert wird. Dabei werden durch den klinisch geschulten Ingenieur bereits Segmente für die Verwendung durch den Chirurgen vorab erstellt. In einer solchen Umgebung kann der Chirurg dann bei der Operationsplanung auf die so vorgeplanten Segmente "zugreifen". Im Rahmen der Besprechung wird seitens des Chirurgen eine gewisse Anzahl von Knochenschnitten und deren Lage besprochen, wobei Anzahl und Lage der Schnitte und die spätere Anordnung in alleiniger Verantwortung und Erfahrung des Chirurgen liegen. Das Verfahren ist daher stark Benutzerabhängig und eine Objektivierung dieses Vorgehens ist nicht möglich.

Zwar kann mit einer Vielzahl von Segmenten ein besseres Ergebnis virtuell erzielt werden, jedoch steigt mit der Anzahl der Knochenschnitte auch die Wahrscheinlichkeit von Knochennekrosen infolge unzureichender metabolischen Austauschs.

Ausgehend hiervon ist es eine Aufgabe der Erfindung ein Operationsplanungssystem zur Verfügung zu stellen, das einen Nachteil oder mehrere Nachteile aus dem Stand der Technik vermeidet und ein verbessertes und/oder einfacheres System zur Verfügung stellt.

### Kurzdarstellung der Erfindung

Die Aufgabe wird gelöst durch ein automatisiertes Operationsplanungssystem für die Rekonstruktion von fehlenden oder geschädigten Knochenteilen, welches eine Einrichtung zum Einlesen von 3D-Bilddaten, wobei die 3D-Bilddaten zumindest ein knöchernes Spenderareal betreffen, Mittel zur Aufbereitung der 3D-Bilddaten in strukturierte Daten, sodass knöcherne Anteile von Weichteilen und/oder Gefäßsystemen unterschieden werden, Mittel zum Einlesen von 3D-Zieldaten betreffend ein fehlendes oder geschädigtes Knochenteil, Mittel zum Erhalt einer oder mehrerer 3D-Zielkurven in Relation zu den 3D-Zieldaten und den 3D-Daten des Spenderareals, Mittel zur Segmentierung der knöchernen Anteile des knöchernen Spenderareals in Segmente, wobei die Mittel zur Segmentierung derart ausgelegt sind, dass die Segmente in den strukturierten 3D-Bilddaten auf Basis der 3D-Zieldaten und einer Anpassung von Abschnitten der 3D-Zielkurve bestimmt werden, und Mittel zum Zusammenfügung der Segmente zu einer Rekonstruktion, wobei die Segmentierung der knöchernen Anteile 3D-Zielkurven an erhaltene 3D-Oberflächen der 3D-Zieldaten betreffend ein fehlendes oder geschädigtes Knochenteil anpasst, aufweist.

Weitere vorteilhafte Ausgestaltungen sind Gegenstand der abhängigen Ansprüche und der ausführlichen Beschreibung sowie der Figuren.

### Kurzdarstellung der Figuren

Nachfolgend wird die Erfindung näher unter Bezugnahme auf in den Figuren gezeigter beispielhafter Ausführungsformen erläutert. In diesen zeigt:
Fig. 1 eine schematische Darstellung eines Zielgebietes,
Fig. 2 eine schematische Darstellung einer Zielkurve,
Fig. 3 eine schematische Darstellung von Segmenten mit einer erzielten Kurve gemäß einer Ausführungsform der Erfindung.
Fig. 4 eine schematische Darstellung von Segmenten eines Spenderareals, welche Verwendung finden in der Darstellung der Figur 3, und
Fig. 5 einen schematischen Vergleich von Zielkurve und erzielbarer Kurve.

### Detaillierte Beschreibung

Nachfolgend wird die Erfindung eingehender unter Bezugnahme auf die Figuren dargestellt werden. Dabei ist anzumerken, dass unterschiedliche Aspekte beschrieben werden, die jeweils einzeln oder in Kombination zum Einsatz kommen können, d.h. jeglicher Aspekt kann mit unterschiedlichen Ausführungsformen der Erfindung verwendet werden, soweit nicht explizit als reine Alternative dargestellt.

Weiterhin wird nachfolgend der Einfachheit halber in aller Regel nur auf eine Entität Bezug genommen werden. Soweit nicht explizit vermerkt, kann die Erfindung aber auch jeweils mehrere der betroffenen Entitäten aufweisen. Insofern ist die Verwendung der Wörter "ein", "eine" und "eines" nur als Hinweis darauf zu verstehen, dass in einer einfachen Ausführungsform zumindest eine Entität verwendet wird.

Gemäß Ausführungsformen der Erfindung wird ein Operationsplanungssystem für die Rekonstruktion von fehlenden oder geschädigten Knochenteilen zur Verfügung gestellt.

Das Operationsplanungssystem weist zumindest eine Einrichtung zum Einlesen von 3D-Bilddaten betreffend zumindest ein knöchernes Spenderareal auf. Beispielsweise können Voxel-Daten direkt von einem entsprechenden Bildgebungsgerät erhalten werden und/oder Voxel-Daten aus (aufbereiteten) zuvor erhobenen Bilddaten eingelesen werden. Hierzu können z.B. geeignete Schnittstellen oder Lesegeräte für Speichermedien vorgesehen sein.

Das Operationsplanungssystem weist zudem Mittel zur Aufbereitung der 3D-Bilddaten in strukturierte Daten auf, sodass knöcherne Anteile von Weichteilen und/oder Gefäßsystemen unterschieden werden. Hierzu kann beispielsweise eine Intensität und/oder eine Intensitätsänderung und/oder Kantenfilter etc. verwendet werden. Hiermit wird es möglich z.B. aus den strukturierten Bilddaten bestimme erkannte Strukturen, z.B. Knochen und/oder Gefäße und/oder muskuläre Anteile und/oder Fettgewebe selektiv darzustellen, selektiv auszuwählen, etc.. Solche Mittel können mittels einer geeignet programmierten Verarbeitungseinheit oder/aber auch in Hardware und/oder einer Mischung aus Hard- und Software bereitgestellt werden.

Das Operationsplanungssystem weist weiterhin Mittel zum Einlesen von 3D-Zieldaten betreffend ein fehlendes oder geschädigtes Knochenteil auf. Beispielsweise können Voxel-Daten direkt von einem entsprechenden Bildgebungsgerät erhalten werden und/oder Voxel-Daten aus (aufbereiteten) zuvor erhaltenen Bilddaten eingelesen werden. Hierzu können z.B. geeignete Schnittstellen oder Lesegeräte für Speichermedien vorgesehen sein. So ist es z.B. möglich Daten durch Spiegelung eines "gesunden" Areals, durch Auswahl aus einer Datenbank, durch manuelle Modellierung und/oder einer Mischung aus diesen zu erhalten. Es sei angemerkt, dass hier auch durch geeignete Nachbearbeitung (Streckung/Stauchung/Drehung) auf individuelle Unterschiede eingegangen werden kann, um z.B. andere Fehlentwicklungen auffangen oder kompensieren zu können und/oder um klinische oder operative Restriktionen zu berücksichtigen.

Das Operationsplanungssystem weist weiterhin Mittel zum Erhalt einer oder mehrerer 3D-Zielkurven in Relation zu den 3D-Zieldaten und den 3D-Daten des Spenderareals auf. Diese Mittel zum Einlesen können z.B. aus den zuvor erhaltenen 3D-Zieldaten aufbereitet sein und/oder interaktiv durch einen Chirurgen, z.B. auf Basis der zuvor erhaltenen 3D-Zieldaten erstellt worden sein. Von besonderem Augenmerk hierbei ist, dass die Zielkurven Kurven im 3D-Raum sind. Durch die Verwendung von Kurven kann in späteren Schritten die Verarbeitung der Daten erheblich vereinfacht und beschleunigt werden.

Das Operationsplanungssystem weist weiterhin Mittel zur Segmentierung der knöchernen Anteile des knöchernen Spenderareals auf. Dabei können auf Basis der 3D-Zieldaten und einer Anpassung von Abschnitten der Zielkurve der 3D-Zieldaten Segmente in den strukturierten 3D-Bilddaten bestimmt werden, die eine gewisse (gewünschte / voreinstellbare) Übereinstimmung aufweisen. Dabei können unterschiedliche Parameter Berücksichtigung finden, wie z.B. minimale Größe eines Segments, maximale Anzahl von Segmenten, maximale Abweichung von der Zielkurve, um nur einige zu nennen. Basierend auf diesen Randbedingungen können unterschiedliche Segmentierungen vorgeschlagen werden, die ggf. anschließend zur weiteren Bewertung durch den Chirurgen aufbereitet werden können. Als ein Implementierungsbeispiel können durch Unschärfe-Operationen, z.B. dem Multiplizieren oder Addieren von zufallswerten, Variationen erstellt werden.

Das Operationsplanungssystem weist weiterhin Mittel zum Zusammenfügung der Segmente zu einer Rekonstruktion auf, wobei die Segmentierung der knöchernen Anteile 3D-Zielkurven an 3D-Oberflächen der 3D-Zieldaten anpasst. Mittels der so erstellten (virtuellen) Rekonstruktion kann der Chirurg basierend auf seiner Erfahrung und Patientenkenntnis eine mögliche Variante auswählen und/oder iterativ nachbearbeiten.

D.h. im Unterschied zu bekannten Verfahren wird nunmehr im erfindungsgemäßen Verfahren die Rezipientenregion explizit mit in Betracht gezogen. Im Unterschied zu bekannten Systemen, bei denen die Planung rechenintensiv anhand von Flächendaten und/oder Volumendaten erfolgt, wird bei dem System gemäß der vorliegenden Erfindung hingegen eine Raumkurve ermittelt, welche sich an anatomischen Verläufen orientieren kann.

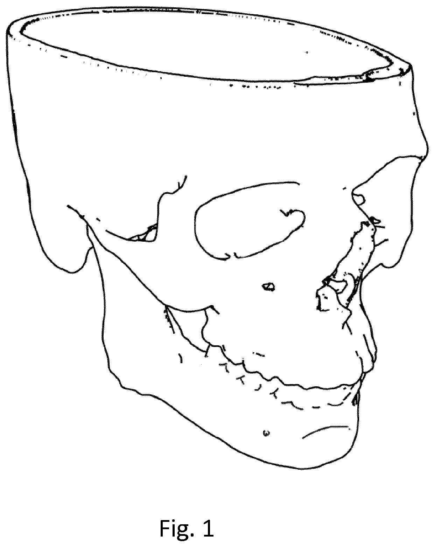

In Figur 1 ist z.B. ein Teil eines Schädels mit einem Unterkiefer dargestellt. Ist nun z.B. ein Teil des Unterkiefers des Patienten geschädigt, so könnte dieser Teil aus anderen knöchernen Bestandteilen rekonstruiert werden.

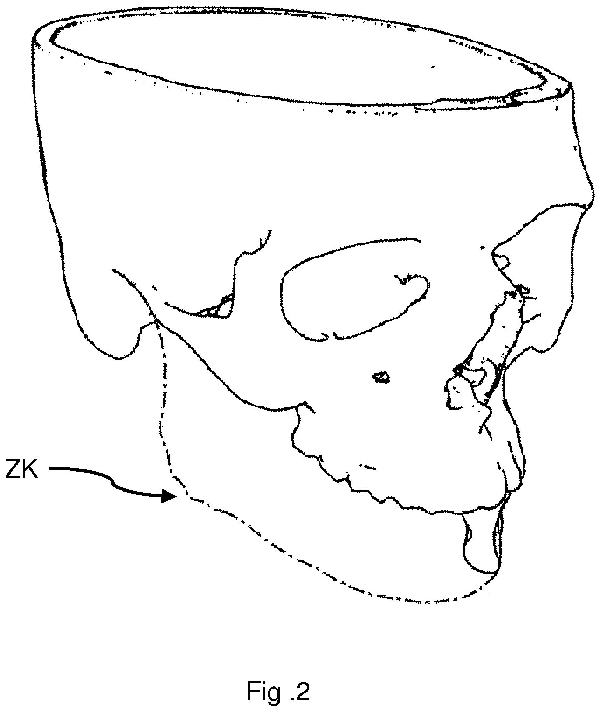

Beispielsweise ist in Figur 2 gestrichelt eine mögliche Zielkurve ZK strichpunktiert dargestellt, die sich z.B. an Hand von früheren Bilddaten und/oder aus einer Bibliothek und/oder aus einer Spiegelung des linken Unterkiefers auf die rechte Seite ergeben kann.

Auch wenn auf einen ersten Blick es so erscheinen mag, dass die Zielkurve ZK im Wesentlichen die äußerste Linie des Zielareals wiedergibt, muss dies nicht immer der Fall sein. Beispielsweise können auch andere anatomische Grenzen, z.B. für Implantate, etc. an einzelnen Punkten oder Abschnitten Grund für die Wahl der Zielkurve sein. Es ist jedoch auch möglich mehr als eine Zielkurve zu definieren. Am Beispiel eines Unterkiefers wäre es z.B. möglich eine erste Zielkurve, die den (ästhetisch) bestimmenden Rand angibt, zu definieren, während eine zweite Zielkurve z.B. ein gewünschtes Profil angibt, das z.B. für Zahn-Implantate vorteilhaft wäre. Andere Parameter, wie z.B. die Berücksichtigung von Gefäßsystemen / Weichteilgewebe können zusätzlich Berücksichtigung finden.

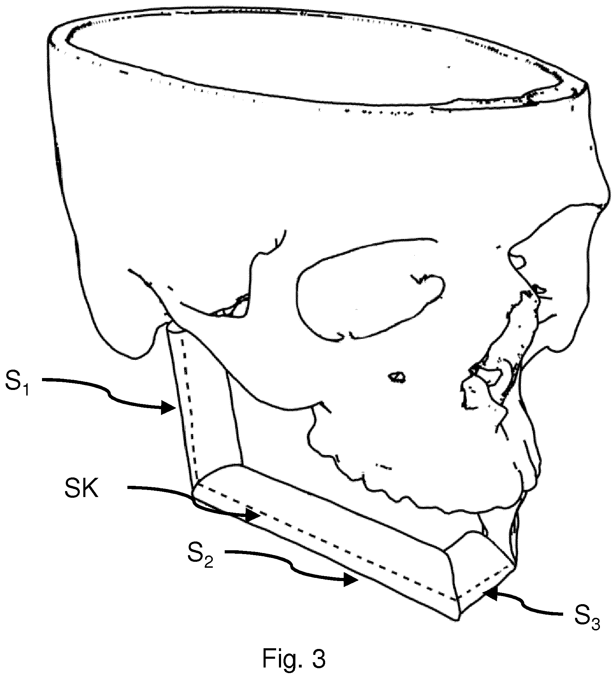

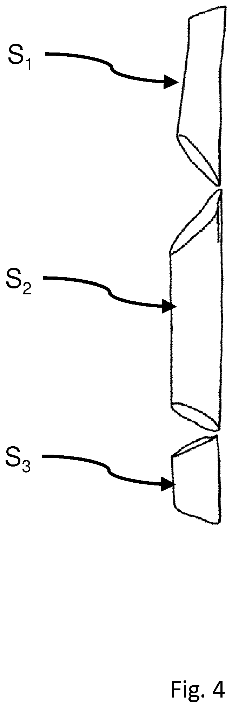

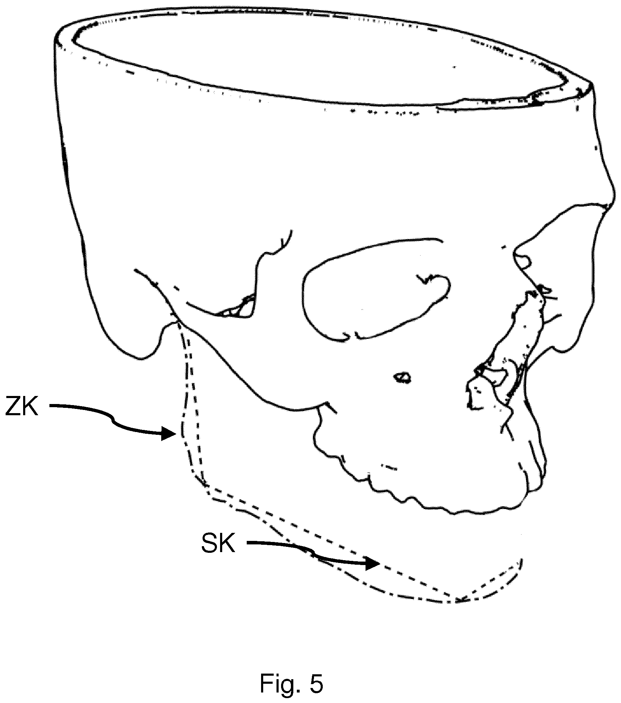

In Figur 3 ist sodann ein Beispiel aufgeführt, wie z.B. aus einem Spenderareal Segmente an die Zielkurve angepasst sind. Beispielsweise ist hier eine Rekonstruktion an Hand von drei Segmenten S₁, S₂, S₃ aufgezeigt. Diese drei Segmente S1, S2, S3 sind aus einem Spenderareal, hier ein Wadenbein gemäß Figur 4, entnommen. Die entsprechende Kurve SK ist hier gestrichelt dargestellt. Der Vergleich zwischen der so erzielbaren gestrichelten Zielkurve SK und der ursprünglichen Zielkurve ZK ist in Figur 5 dargestellt.

D.h. die Erfindung betrifft ein System und ein Verfahren zur Planung von Knochenrekonstruktionen mit autologen oder allologen Transplantaten, bei dem aus donor- und rezipientenseitig gewonnenen 3D-Daten mittels Triangulation Oberflächendarstellungen der Donor- und der Rezipientenregion gewonnen werden und jeweils wenigstens eine (Kontur-) Linie auf der donor- und rezipientenseitigen Oberfläche definiert werden, wobei die (Kontur-) Linie anhand der Krümmung der jeweiligen Oberfläche an das Transplantat und die Rekonstruktion angepasst werden kann.

Das entwickelte System und Verfahren ermöglicht die computergestützte Planung von knöchernen Rekonstruktionen, insbesondere im gesichtschirurgischen Bereich.

Die explizite Neuerung des Verfahrens ist die Verwendung von drei-dimensionalen Kurven, die sich an den individuellen knöchernen Geometrien des Patienten orientieren. Diese Kurven werden in der Regel sowohl an dem Transplantatknochen als auch an der geplanten Empfängerregion entweder manuell oder automatisch (computergestützt) erzeugt und bieten dadurch die Möglichkeit einer Vereinfachung der komplexen dreidimensionalen Oberflächengeometrie der Knochen. Durch diese Vereinfachung wird eine zumindest teilweise automatisierte Planung der Rekonstruktion ermöglicht. Auf dieser Basis können ggf. Schnittschablonen und individualisierte Verplattungssysteme erstellt werden, welche die Durchführung der Operation vereinfachen sowie beschleunigen und so zu einem verbesserten Operationsergebnis führen können.

In einer Ausführungsform der Erfindung weist die Planung einstellbare minimale Größen für die Segmente auf. Beispielsweise kann so verhindert werden, dass Segmente vorgeschlagen werden, die bei der Trennung so klein sind, dass die vaskulare Versorgung nicht sichergestellt ist und die Gefahr von Nekrosen besteht.

In einer weiteren Ausführungsform der Erfindung weist die Planung eine einstellbare maximale Größe für die Segmente auf. Wird die Größe eines Segmentes zu groß gewählt besteht im Allgemeinen die Gefahr, dass die Abweichung von der Zielkurve groß wird.

In einer weiteren Ausführungsform der Erfindung kann das das System weiterhin Mittel zur Erstellung von Strukturdaten für eine Operations-Schablone zum Einsatz bei einer nachfolgenden Operation aufweisen. Beispielsweise kann eine solche Schablone ein Spenderareal fixieren und Schnittkanten vorgeben, sodass die Segmente entsprechend der (ausgewählten) Segmentierung erstellt werden können. Beispielsweise können entsprechende Schnittebenen durch die Schablone vorgegeben sein, sodass die Segmente (wie in Figur 4 gezeigt) aus einem beispielhaften Wadenbein erstellt werden können.

Ohne Weiteres können die Strukturdaten für eine Operations-Schablone Strukturdaten für einen 3D-Drucker sein. In diesem Fall ist es möglich, eine solche Schablone durch einen spezialisierten Hersteller oder aber lokal vor Ort zu erstellen.

Von besonderem Vorteil ist es, dass das System auf Grund der Strukturierung der 3D-Bilddaten es zulässt, dass Segmentierung und Planung auch Weichteile und/oder Gefäßsysteme berücksichtigen. Beispielsweise kann so bereits zum Zeitpunkt der Planung ein eventueller Weichteilanteil berücksichtigt werden, der bei der Rekonstruktion ästhetisch von Vorteil ist. Ebenso können alternativ oder zusätzlich bereits Gefäße berücksichtigt werden, die zur nachfolgenden Versorgung von Knochen und anderem Gewebe nötig sind. Werden diese bereits aus dem Spenderareal berücksichtigt, kann die Operationszeit verkürzt werden. Kürzere Operationszeiten wirken sich im Allgemeinen positiv auf die Heilung aus.

Ohne Weiteres kann durch das erfindungsgemäße Operationsplanungssystem eine Mehrzahl von Vorschlägen für eine Segmentierung an Hand leicht zu variierender Randbedingungen erstellt werden, wobei ein, mehrere oder alle Vorschläge visualisiert zur Auswahl zur Verfügung gestellt werden. D.h. der Chirurg kann aus den Vorschlägen auswählen, was für den Patienten am sinnvollsten erscheint. So kann es z.B. sein, dass mit einem Segment mehr bei nur wenig längerer Operationszeit eine funktional und/oder ästhetisch bessere Rekonstruktion ermöglicht wird. Dann könnte der Chirurg, wenn er es für angezeigt hält, diesen etwas umfangreicheren Vorschlag auswählen. Des Weiteren kann auch eine computergestützte Quantifizierung der Übereinstimmung zwischen Planung und Zielgeometrie zur Verfügung gestellt werden, welche in die Entscheidungsfindung mit einfließen kann.

Gemäß einer weiteren Ausgestaltung der Erfindung stellt das Operationsplanungssystem zudem Mittel zur gleichzeitigen Visualisierung der 3D-Zieldaten und der durch Segmentierung erstellten Rekonstruktion zur Verfügung. Ein Beispiel einer solchen Visualisierung ist in Figur 5 gezeigt. Visualisierungen können z.B. auf einem Monitor oder einem Virtual Reality System zur Verfügung gestellt werden.

Gemäß einer weiteren Ausgestaltung der Erfindung können die 3D-Bilddaten Daten einer Computer Tomographie und/oder eines MRT, MRT-Angio und/oder eines PET/CT, SPECT/CT, CT-Angio, Digitale Volumentomographie, 3D-Sonographie sein, ohne jedoch hierauf beschränkt zu sein. Offensichtlich können auch "fusionierte" Bilddaten aus unterschiedlichen Verfahren zum Einsatz kommen und/oder Strukturdaten aus anderen Software-Tools.

Gemäß noch einer weiteren Ausgestaltung der Erfindung werden die 3D-Zieldaten durch Kopie und/oder Spiegelung und/oder relativer Repositionierung von korrespondierenden fehlenden oder geschädigten Knochenteilen desselben Patienten aufweisen oder aus einer anthropometrischen Geometriedatenbank generiert werden. Eine relative Repositionierung kann z.B. bei Trümmerbrüchen von Vorteil sein.

Ohne Beschränkung der Allgemeinheit der Erfindung ist es auch möglich aus dem Operationsplanungssystem heraus Daten für eine roboter-unterstützte Operation und/oder echtzeitnavigierte Chirurgie zur Verfügung zu stellen. D.h., Daten aus dem Operationsplanungssystem können bei der Operation, insbesondere bei der Segmentierung des Spenderareals aber auch bei der Repositionierung im Zielareal verwendet werden, um z.B. eine möglichst präzise Operationsführung zu ermöglichen. Hierbei können Daten in Bildsysteme eingespeist werden und/oder Positionierungsaufgaben unterstützt werden.

Von besonderem Vorteil ist, dass das vorgestellte erfindungsgemäße Operationsplanungssystem vielseitig anwendbar ist. So erlaubt das System, dass die fehlenden oder geschädigten Knochenteile Teile entweder einer Mandibula, einer Maxilla, eines Zygomas, eines Craniums, eines Handknochens, eines Fußknochens, einer Tibia, eines Humerus, eines Femurs, eines Radius oder einer Ulna sind.

Von besonderem Vorteil ist, dass das vorgestellte erfindungsgemäße Operationsplanungssystem vielseitig anwendbar ist. So erlaubt das System, dass das knöcherne Spenderareal ein Beckenkamm und/oder eine Scapula und/oder eine Fibula und/oder ein Radius und/oder ein Femur und/oder eine Rippe und/oder ein Cranium und / oder eine Tabula externa ist.

Ohne weiteres kann das System aber auch unterschiedliche Spenderareale für ein Zielareal berücksichtigen. So kann z.B. untersucht werden, ob ein alternatives Spenderareal oder eine Mischung von Segmenten aus unterschiedlichen Spenderarealen unter Umständen ein besseres Ergebnis zur Verfügung stellt.

Mit dem vorgestellten Verfahren kann nunmehr auf relativ kostengünstige Hardware zurückgegriffen werden, da die Anforderungen an die Rechenleistung durch geringere Anforderungen sinken. Da zudem das Spenderareal als auch das Zielareal berücksichtigt werden, ist das Ergebnis vergleichbar oder besser zu bisherigen Systemen. Zugleich kann aber durch die zusätzlich mögliche Berücksichtigung von anderen Geweben / Gefäßen auf Grund der Strukturierung das Gesamtergebnis wesentlich verbessert werden.

Dabei ist durch das Operationsplanungssystem eine weitgehende Automatisierung ermöglicht, sodass Erfahrungswerte als Randwerte Berücksichtigung finden und so eine verbesserte Auswahl auch bei weniger erfahrenen Chirurgen ermöglichen.

Insbesondere kann das System aufzeigen, welche Teile eines Spenderareals wie zu verwenden sind, in dem Anzahl, Ort und Richtung von Knochenschnitten und damit auch die Anzahl der verwertbaren Segmente bestimmt werden kann. Dabei kann die Komplexität so gering gehalten werden, dass mit herkömmlicher Rechnerhardware zeitnah eine Operationsplanung durchgeführt werden kann. Dies wird dadurch ermöglicht, dass das geometrisch schlecht-konditionierte Problem der Anordnung von Flächen mit unterschiedlichen Krümmungen auf die Anordnung von räumlichen Kurven reduziert wurde. Hierdurch werden auch Benutzerinteraktionen in Bezug auf Parameter / Grenzwerte ermöglicht.

## Patentansprüche

1. Automatisiertes Operationsplanungssystem für die Rekonstruktion von fehlenden oder geschädigten Knochenteilen, aufweisend
• eine Einrichtung zum Einlesen von 3D-Bilddaten,
• Mittel zum Einlesen von 3D-Zieldaten betreffend ein fehlendes oder geschädigtes Knochenteil,
wobei
• die 3D-Bilddaten zumindest ein knöchernes Spenderareal betreffen und das automatisierte Operationsplanungssystem weiterhin aufweist
• Mittel zur Aufbereitung der 3D-Bilddaten in strukturierte Daten, sodass knöcherne Anteile von Weichteilen und/oder Gefäßsystemen unterschieden werden,
• Mittel zum Erhalt einer oder mehrerer 3D-Zielkurven in Relation zu den 3D-Zieldaten und den 3D-Daten des Spenderareals,
• Mittel zur Segmentierung der knöchernen Anteile des knöchernen Spenderareals in Segmente, wobei die Mittel zur Segmentierung derart ausgelegt sind, dass die Segmente in den strukturierten 3D-Bilddaten auf Basis der 3D-Zieldaten und einer Anpassung von Abschnitten der 3D-Zielkurve bestimmt werden,
• Mittel zum Zusammenfügung der Segmente zu einer Rekonstruktion, wobei die Segmentierung der knöchernen Anteile erhaltene 3D-Zielkurven an 3D-Oberflächen der 3D-Zieldaten betreffend ein fehlendes oder geschädigtes Knochenteil anpasst.

2. Operationsplanungssystem nach Anspruch 1, **dadurch gekennzeichnet, dass** die Segmentierung einstellbare minimale Größen für die Segmente aufweist.

3. Operationsplanungssystem nach Anspruch 1 oder 2, **dadurch gekennzeichnet, dass** die Segmentierung einstellbare maximale Größen für die Segmente aufweist.

4. Operationsplanungssystem nach einem der vorhergehenden Ansprüche, **dadurch gekennzeichnet, dass** das System weiterhin Mittel zur Erstellung von Strukturdaten für eine Operations-Schablone zum Einsatz bei einer nachfolgenden Operation aufweist.

5. Operationsplanungssystem nach Anspruch 4, **dadurch gekennzeichnet, dass** die Strukturdaten für eine Operations-Schablone Strukturdaten für einen 3D-Drucker sind.

6. Operationsplanungssystem nach einem der vorhergehenden Ansprüche, **dadurch gekennzeichnet, dass** Segmentierung und Operationsplanung auch Weichteile und/oder Gefäßsysteme berücksichtigen.

7. Operationsplanungssystem nach einem der vorhergehenden Ansprüche, **dadurch gekennzeichnet, dass** eine Mehrzahl von Vorschlägen für eine Segmentierung an Hand leicht zu variierender Randbedingungen erstellt wird, wobei ein, mehrere oder alle Vorschläge visualisiert zur Auswahl zur Verfügung gestellt werden kann.

8. Operationsplanungssystem nach einem der vorhergehenden Ansprüche, **dadurch gekennzeichnet, dass** zudem Mittel zur gleichzeitigen Visualisierung der 3D-Zieldaten und der durch Segmentierung erstellten Rekonstruktion zur Verfügung gestellt werden.

9. Operationsplanungssystem nach einem der vorhergehenden Ansprüche, **dadurch gekennzeichnet, dass** die 3D-Bilddaten Daten einer Computer Tomographie und/oder eines MRT, MRT-Angio und/oder eines PET/CT, SPECT/CT, CT-Angio, Digitale Volumentomographie, 3D-Sonographie sind.

10. Operationsplanungssystem nach einem der vorhergehenden Ansprüche, **dadurch gekennzeichnet, dass** die 3D-Zieldaten durch Kopie und/oder Spiegelung und/oder relativer Repositionierung von korrespondierenden fehlenden oder geschädigten Knochenteilen desselben Patienten aufweisen oder aus einer anthropometrischen Geometriedatenbank generiert werden können.

11. Operationsplanungssystem nach einem der vorhergehenden Ansprüche, **dadurch gekennzeichnet, dass** Daten für eine roboter-unterstützte Operation und/oder echtzeitnavigierte Chirurgie zur Verfügung gestellt werden können.

12. Verwendung eines Systems nach einem der vorhergehenden Ansprüche, wobei die fehlenden
oder geschädigten Knochenteile als 3D-Zieldaten eingelesen werden und Teile entweder einer Mandibula, einer Maxilla, eines
Zygomas, eines Craniums, eines Handknochens, eines Fußknochens, einer Tibia, eines Humerus, eines Femurs, eines Radius oder einer Ulna sind.

13. Verwendung eines Systems nach einem der vorhergehenden Ansprüche 1 bis 11, wobei das
knöcherne Spenderareal als 3D-Bilddaten eingelesen wird und entweder ein Beckenkamm und/oder eine Scapula und/oder eine Fibula
und/oder ein Radius und/oder ein Femur und/oder eine Rippe und/oder ein Cranium und / oder eine Tabula externa ist.

14. Verwendung eines Systems nach Anspruch 12, wobei das knöcherne
Spenderareal als 3D-Bilddaten eingelesen wird und entweder ein Beckenkamm und/oder eine Scapula und/oder eine Fibula und/oder ein
Radius und/oder ein Femur und/oder eine Rippe und/oder ein Cranium und / oder eine Tabula externa ist.

## Claims

1. Automated surgical planning system for the reconstruction of missing or damaged bone parts, comprising
• a device for reading in of 3D image data,
• means for reading in of 3D target data relating to a missing or damaged bone part, whereby
• the 3D image data relate to an osseous donor area and the automated surgical planning system further comprises
• means for processing the 3D image data into structured data, so that osseous portions are distinguished from soft tissues and/or vascular systems,
• means for obtaining one or more 3D target curves in relation to the 3D target data and the 3D data of the donor area,
• means for segmentation of the osseous portions of the osseous donor area in segments, whereby the means for segmentation are arranged such that the segments within the structured 3D image data are determined on basis of the 3D target data and an adaption of sections of the 3d target curve,
• means for merging the segments into a reconstruction, wherein the segmentation of the osseous portions adapts 3D target curves to 3D surfaces of the 3D target data relating to a missing or damaged bone part.

2. Surgical planning system according to claim 1, **characterized in that** the segmentation comprises adjustable minimum sizes for the segments.

3. Surgical planning system according to claim 1 or 2, **characterized in that** the segmentation comprises adjustable maximum sizes for the segments.

4. Surgical planning system according to one of the preceding claims, **characterized in that** the system furthermore comprises means of creating structural data for a surgical template for use in a subsequent surgery.

5. Surgical planning system according to claim 4, **characterized in that** the structural data for a surgical template is structural data for a 3D printer.

6. Surgical planning system according to one of the preceding claims, **characterized in that** the segmentation and surgery planning take soft tissues and/or vascular systems into consideration.

7. Surgical planning system according to one of the preceding claims, **characterized in that** a number of proposals are created for a segmentation by means of easily variable boundary conditions, and one, several, or all of the proposals are visualized for a selection.

8. Surgical planning system according to one of the preceding claims, **characterized in that** means are furthermore provided for the simultaneous visualization of the 3D target data and the reconstruction produced by segmentation.

9. Surgical planning system according to one of the preceding claims, **characterized in that** the 3D image data is data from computer tomography and/or MRT, MRT-angio and/or PET/CT, SPECT/CT, CT-angio, digital volume tomography, and 3D sonography.

10. Surgical planning system according to one of the preceding claims, **characterized in that** the 3D target data comprises copying and/or mirroring and/or relative repositioning of corresponding missing or damaged bone parts of the same patient or is generated from an anthropometric geometry database.

11. Surgical planning system according to one of the preceding claims, **characterized in that** data is provided for a robot-assisted surgery and/or real-time navigated surgery.

12. Use of a system according to one of the preceding claims, wherein the missing or damaged bone parts are read in as 3D target data and are parts of either a mandibula, a maxilla, a zygoma, a cranium, a hand bone, a foot bone, a tibia, a humerus, a femur, a radius or an ulna.

13. Use of a system according to one of the preceding claims 1 to 11, wherein the osseous donor area is read in as 3D image data and is an iliac crest and/or a scapula and/or a fibula and/or a radius and/or a femur and/or a rib and/or a cranium and/or a tabula externa.

14. Use of a system according to claim 12, wherein the osseous donor area is read in as 3D image data and either is an iliac crest and/or a scapula and/or a fibula and/or a radius and/or a femur and/or a rib and/or a cranium and/or a tabula externa.

## Revendications

1. Système automatisé de programmation opératoire, pour la reconstruction de parties osseuses manquantes ou détériorées, comportant
• un système de lecture de données d'images en 3D,
• des moyens de lecture de données-cibles en 3D concernant une partie osseuse manquante ou détériorée,
• les données d'images en 3D concernant au moins un site osseux donneur et le système automatisé de programmation des opérations comportant en outre
• des moyens de préparation des données d'images en 3D en des données structurelles, de sorte que la distinction soit faite avec des fractions osseuses de parties molles et/ou des systèmes vasculaires,
• des moyens d'obtention de plusieurs courbes cibles en 3D, en relation avec les données cibles en 3D et les données en 3D du site donneur,
• des moyens de segmentation des fractions osseuses du site osseux donneur en segments, les moyens de segmentation étant conçus de sorte que les segments dans les données d'images en 3D structurées soient déterminés sur la base des données cibles en 3D et d'une adaptation de fractions de la courbe cible en 3D,
• des moyens d'assemblage des segments en une reconstruction, la segmentation des fractions osseuses adaptant des courbes cibles en 3 D obtenues à des surfaces en 3D des données cibles en 3D en ce qui concerne une partie osseuse manquante ou détériorée.

2. Système de programmation des opérations selon la revendication 1, **caractérisé en ce que** la segmentation comporte des grandeurs minimales réglables pour les segments.

3. Système de programmation des opérations selon la revendication 1 ou 2, **caractérisé en ce que** la segmentation comporte des grandeurs maximales réglables pour les segments.

4. Système de programmation des opérations selon l'une quelconque des revendications précédentes, **caractérisé en ce que** le système comporte en outre des moyens d'établissement de données structurelles pour un gabarit opératoire, destiné à l'utilisation lors d'une opération suivante.

5. Système de programmation des opérations selon la revendication 4, **caractérisé en ce que** les données structurelles pour un gabarit opératoire sont des données structurelles pour une imprimante en 3D.

6. Système de programmation des opérations selon l'une quelconque des revendications précédentes, **caractérisé en ce que** la segmentation et la programmation opératoire prennent également en compte des parties molles et/ou des systèmes vasculaires.

7. Système de programmation des opérations selon l'une quelconque des revendications précédentes, **caractérisé en ce qu'**une multiplicité de propositions pour une segmentation est établie à l'aide de conditions cadres facilement variables, une, plusieurs ou toutes les propositions pouvant être mise(s) à disposition sous forme visualisée pour leur sélection.

8. Système de programmation des opérations selon l'une quelconque des revendications précédentes, **caractérisé en ce que** de surcroît, des moyens de visualisation simultanée des données cibles en 3D et de la reconstruction établie par segmentation sont mis à disposition.

9. Système de programmation des opérations selon l'une quelconque des revendications précédentes, **caractérisé en ce que** les données d'images en 3D sont des données d'une tomodensitométrie et/ou d'une IRM, d'une angio-IRM et/ou d'un scanner CT, d'un SPECT/CT, d'un angio-CT, d'une tomographie volumétrique numérisée, d'une sonographie en 3D.

10. Système de programmation des opérations selon l'une quelconque des revendications précédentes, **caractérisé en ce que** les données cibles en 3D pourront être générées par copie et/ou par réflexion et/ou par repositionnement relatif de parties osseuses manquantes ou détériorées correspondantes du même patient ou à partir d'une base de données anthropométrique de géométrie.

11. Système de programmation des opérations selon l'une quelconque des revendications précédentes, **caractérisé en ce que** des données pour une opération assistée par robot et/ou pour une chirurgie par navigation en temps réel peuvent être mises à disposition.

12. Utilisation d'un système selon l'une quelconque des revendications précédentes, les parties osseuses manquantes ou détériorées étant lues sous la forme de données cibles en 3D et étant des parties soit d'un maxillaire inférieur, d'un maxillaire supérieur, d'un zygoma, d'un crâne, d'un os de la main, d'un os du pied, d'un tibia, d'un humérus, d'un fémur, d'un radius ou d'un cubitus.

13. Utilisation d'un système selon l'une quelconque des revendications précédentes 1 à 11, le site osseux donneur étant lu sous la forme de données d'images en 3D et étant soit une crête iliaque et/ou une omoplate et/ou un péroné et/ou un radius et/ou un fémur et/ou une côte et/ou un crâne et/ou une lame externe.

14. Utilisation d'un système selon la revendication 12, le site osseux donneur étant lu sous la forme de données d'images en 3D et étant soit une crête iliaque et/ou une omoplate et/ou un péroné et/ou un radius et/ou un fémur et/ou une côte et/ou un crâne et/ou une lame externe.
